# EUROPEAN PATENT APPLICATION

(11) **EP 4 807 009 A1**
(43) Date of publication of application: **16.09.2026**
(21) Application number: 24889990.8
(22) Date of filing: 06.05.2024
(51) Int. Cl.: C12N 15/81, C12N 1/19

(54) **METHOD FOR RECOMBINANTLY EXPRESSING FELINE SERUM ALBUMIN**

(30) Priority: 13.11.2023 CN 202311503698
(71) Applicant: Beijing Shunlei Tecnology Co., Ltd., Beijing 100090 (CN)
(72) Inventor: DOU, Xin, Beijing 100090 (CN); ZHAN, Shunli, Beijing 100090 (CN); GAO, Lei, Beijing 100090 (CN); MO, Wenyi, Beijing 100090 (CN); JIA, Ning, Beijing 100090 (CN)
(74) Representative: Winter, Brandl - Partnerschaft mbB
(86) International application number: PCT/CN2024/091170
(87) International publication number: WO 2025/102619

(57) **Abstract**

The present disclosure discloses a method for recombinant expression of feline serum albumin (FSA). This disclosure provides a method for constructing a recombinant engineering strain for expressing feline serum albumin, which involves co-expressing feline serum albumin, protein disulfide isomerase, and the regulatory factors HAC1 and/or Vitreoscilla hemoglobin in a recipient yeast strain. The resulting strain is the engineering strain for expressing recombinant feline serum albumin (rFSA). The engineering strain of this disclosure can efficiently express rFSA. Compared to the direct and separate expression of rFSA, the expression level of rFSA in the co-expressing strain is significantly increased, reaching up to approximately 16 g/L. This disclosure lays the foundation for large-scale industrial production of rFSA.

## Description

### CROSS-REFERENCE TO RALATED APPLICATIONS

This application claims priority to the Chinese patent application No. 202311503698.4 filed on November 13, 2023, the entire contents of which are hereby incorporated by reference into this document.

### TECHNICAL FIELD

The present disclosure pertains to the field of biotechnology, specifically relating to a method for recombinant expression of feline serum albumin.

### BACKGROUND

Feline Serum Albumin (FSA) is the most abundant protein in feline serum, performing various physiological functions such as maintaining osmotic pressure and transporting endogenous and exogenous substances. Clinically, serum albumin is used to treat conditions such as shock caused by blood loss or burns, hypoproteinemia, and edema resulting from liver cirrhosis and kidney diseases in cats. The primary source of serum albumin is through blood extraction; however, the scarcity of feline serum itself, coupled with the risk of bloodborne virus transmission, makes it unable to meet the market demand for FSA. Some pet hospitals use bovine or human serum albumin as substitutes for FSA in treatment, which is extremely costly and can trigger allergic reactions. Therefore, exploring new production methods for FSA is essential to meet the growing market demand.

Pichia pastoris is a commonly used genetic engineering expression system that enables high-density fermentation, rapid reproduction, and post-translational modification and processing of proteins, making it widely applied in exogenous protein expression. Currently, research on expressing human serum albumin using Pichia pastoris is relatively mature, but reports on recombinant expression of FSA are scarce. Chinese patent CN111118018B discloses a method for expressing FSA in Pichia pastoris, yet the yield is only 2 g/L, which fails to meet the requirements for industrial production of FSA. Thus, there is a need to find new methods to construct more productive strains for FSA production.

FSA is a single-chain protein composed of 584 amino acids, featuring 17 pairs of disulfide bonds and three helical domains, making its structure complex. When expressing FSA in Pichia pastoris, incorrect disulfide bond pairing may occur, leading to the accumulation of a large number of improperly folded nascent proteins in the endoplasmic reticulum of Pichia pastoris. This triggers the unfolded protein response (UPR), thereby reducing the yield of FSA.

### SUMMARY

The objective of this disclosure is to provide a method for recombinant expression of feline serum albumin (FSA).

This disclosure claims protection for a method of constructing an engineering strain for recombinant expression of feline serum albumin.

The method of constructing an engineering strain for recombinant expression of feline serum albumin claimed by this disclosure can be any of the following:
Method I: It may include the following step (A):
   (A) co-expressing feline serum albumin (FSA), protein disulfide isomerase (PDI), and regulatory factor HAC1 in a recipient yeast strain, with the resulting strain designated as Engineering Strain 1; the Engineering Strain 1 is an engineering strain for recombinant expression of feline serum albumin.
Method II: It may include the following step (B):
   (B) co-expressing feline serum albumin (FSA), protein disulfide isomerase (PDI), and Vitreoscilla hemoglobin (VHb) in a recipient yeast strain, with the resulting strain designated as Engineering Strain 2; the Engineering Strain 2 is an engineering strain for recombinant expression of feline serum albumin.
Method III: It may include the following step (C):
   (C) co-expressing feline serum albumin (FSA), protein disulfide isomerase (PDI), regulatory factor HAC1, and Vitreoscilla hemoglobin (VHb) in a recipient yeast strain, with the resulting strain designated as Engineering Strain 3; the Engineering Strain 3 is an engineering strain for recombinant expression of feline serum albumin.

Further, step (A) may involve: introducing the coding gene for serum albumin (FSA), the coding gene for protein disulfide isomerase (PDI), and the coding gene for regulatory factor HAC1 into the recipient yeast strain, with the resulting strain being Engineering Strain 1.

Further, step (B) may involve: introducing the coding gene for serum albumin (FSA), the coding gene for protein disulfide isomerase (PDI), and the coding gene for Vitreoscilla hemoglobin (VHb) into the recipient yeast strain, with the resulting strain being Engineering Strain 2.

Further, step (C) may involve: introducing the coding gene for serum albumin (FSA), the coding gene for protein disulfide isomerase (PDI), the coding gene for regulatory factor HAC1, and the coding gene for Vitreoscilla hemoglobin (VHb) into the recipient yeast strain, with the resulting strain being Engineering Strain 3.

Further still, in the aforementioned steps (A), (B), and (C), the respective coding genes introduced into the recipient yeast strain are introduced in the form of recombinant yeast expression vectors. After introduction, the process may also include enriching the resulting recombinant strains using antibiotics corresponding to the resistance genes carried on the recombinant yeast expression vectors (e.g., secondary enrichment, with the second antibiotic concentration higher than the first). The purpose of this step is to obtain multi-copy integrated strains, with theoretically higher antibiotic concentrations leading to higher integration copy numbers (refer to the Pichia pastoris expression manual for detailed information).

Specifically, step (A) may include: (A1) introducing the coding gene for serum albumin (FSA) into the recipient yeast strain using the yeast expression vector pPIC9K, and enriching the resulting recombinant strains with G418 (e.g., secondary enrichment, with the first G418 concentration at 2 mg/ml and the second at 4 mg/ml) to obtain a multi-copy integrated rFSA expression strain GS115-FSA; (A2) introducing the coding gene for protein disulfide isomerase (PDI) into GS115-FSA using the yeast expression vector pPICZα, and enriching the resulting recombinant strains with zeocin (e.g., secondary enrichment, with the first zeocin concentration at 2 mg/ml and the second at 4 mg/ml) to obtain an rFSA expression strain GS115-FSA-PDI with multi-copy integration of PDI; and (A3) introducing the coding gene for regulatory factor HAC1 into GS115-FSA-PDI using the yeast expression vector pPIC6α, and enriching the resulting recombinant strains with Blasticidin (e.g., secondary enrichment, with the first Blasticidin concentration at 2 mg/ml and the second at 4 mg/ml) to obtain an rFSA expression strain GS115-FSA-PDI-HAC1 with multi-copy integration of HAC1, which is Engineering Strain 1.

Specifically, step (B) may include: (B1) the same as step (A1); (B2) the same as step (A2); (B3) introducing the coding gene for Vitreoscilla hemoglobin (VHb) together with the yeast expression vector pPIC6α into GS115-FSA-PDI, and enriching the resulting recombinant strains with Blasticidin (e.g., secondary enrichment, with the first Blasticidin concentration at 2 mg/ml and the second at 4 mg/ml) to obtain an rFSA expression strain GS115-FSA-PDI-VHb with multi-copy integration of VHb, which is Engineering Strain 2.

Specifically, step (C) may include: (C1) the same as step (A1); (C2) the same as step (A2); (C3) introducing the coding gene for regulatory factor HAC1 and the coding gene for Vitreoscilla hemoglobin (VHb) into GS115-FSA-PDI using the yeast expression vector pPIC6α, and enriching the resulting recombinant strains with Blasticidin (e.g., secondary enrichment, with the first Blasticidin concentration at 2 mg/ml and the second at 4 mg/ml) to obtain an rFSA expression strain GS115-FSA-PDI-HAC1-VHb with multi-copy integration of both HAC1 and VHb, which is Engineering Strain 3.

In the aforementioned methods, feline serum albumin (FSA) may be a protein with the amino acid sequence SEQ ID No.1, or a protein with the same function that has undergone substitution, deletion, and/or addition of one or several amino acid residues from SEQ ID No.1, or a protein with the same function that shares more than 99%, 95%, 90%, 85%, or 80% homology with SEQ ID No.1, or a fusion protein obtained by attaching a tag to the N-terminus and/or C-terminus of the protein with the amino acid sequence SEQ ID No.1.

In the aforementioned methods, protein disulfide isomerase (PDI) may be a protein with the amino acid sequence SEQ ID No.5, or a protein with the same function that has undergone substitution, deletion, and/or addition of one or several amino acid residues from SEQ ID No.5, or a protein with the same function that shares more than 99%, 95%, 90%, 85%, or 80% homology with SEQ ID No.5, or a fusion protein obtained by attaching a tag to the N-terminus and/or C-terminus of the protein with the amino acid sequence SEQ ID No.5.

In the aforementioned methods, regulatory factor HAC1 may be a protein with the amino acid sequence SEQ ID No.9, or a protein with the same function that has undergone substitution, deletion, and/or addition of one or several amino acid residues from SEQ ID No.9, or a protein with the same function that shares more than 99%, 95%, 90%, 85%, or 80% homology with SEQ ID No.9, or a fusion protein obtained by attaching a tag to the N-terminus and/or C-terminus of the protein with the amino acid sequence SEQ ID No.9.

In the aforementioned methods, Vitreoscilla hemoglobin (VHb) may be a protein with the amino acid sequence SEQ ID No.13, or a protein with the same function that has undergone substitution, deletion, and/or addition of one or several amino acid residues from SEQ ID No.13, or a protein with the same function that shares more than 99%, 95%, 90%, 85%, or 80% homology with SEQ ID No.13, or a fusion protein obtained by attaching a tag to the N-terminus and/or C-terminus of the protein with the amino acid sequence SEQ ID No.13.

Here, the substitution, deletion, and/or addition of one or several amino acid residues refer to no more than ten amino acid residues.

Among the aforementioned proteins, a tag refers to a polypeptide or protein fused and expressed with the target protein using DNA in vitro recombination technology to facilitate the expression, detection, tracing, and/or purification of the target protein. The tag may be a Flag tag, His tag, MBP tag, HA tag, myc tag, GST tag, and/or SUMO tag, etc.

Among the aforementioned proteins, homology refers to the identity of amino acid sequences. The identity of amino acid sequences can be determined using homology search sites on the internet, such as the BLAST webpage on the NCBI homepage. For example, in advanced BLAST 2.1, by using blastp as the program, setting the Expect value to 10, turning off all Filters, using BLOSUM 62 as the Matrix, and setting the Gap existence cost, Per residue gap cost, and Lambda ratio to 11, 1, and 0.85 (default values), respectively, and performing the search, the identity value (%) of a pair of amino acid sequences can be calculated.

Among the aforementioned proteins, homology of more than 95% may indicate at least 96%, 97%, or 98% identity. Homology of more than 90% may indicate at least 91%, 92%, 93%, or 94% identity. Homology of more than 85% may indicate at least 86%, 87%, 88%, or 89% identity. Homology of more than 80% may indicate at least 81%, 82%, 83%, or 84% identity.

In the aforementioned methods, the coding gene for serum albumin (FSA) may be a DNA molecule with the nucleotide sequence SEQ ID No.2, or a DNA molecule that hybridizes with the DNA molecule shown in SEQ ID No.2 under stringent conditions and encodes a protein with the amino acid sequence SEQ ID No.1, or a DNA molecule that shares more than 99%, 95%, 90%, 85%, or 80% homology with the DNA sequence defined by SEQ ID No.2 and encodes a protein with the amino acid sequence SEQ ID No.1.

In the aforementioned methods, the coding gene for protein disulfide isomerase (PDI) may be a DNA molecule with the nucleotide sequence SEQ ID No.6, or a DNA molecule that hybridizes with the DNA molecule shown in SEQ ID No.6 under stringent conditions and encodes a protein with the amino acid sequence SEQ ID No.5, or a DNA molecule that shares more than 99%, 95%, 90%, 85%, or 80% homology with the DNA sequence defined by SEQ ID No.6 and encodes a protein with the amino acid sequence SEQ ID No.5.

In the aforementioned methods, the coding gene for regulatory factor HAC1 may be a DNA molecule with the nucleotide sequence SEQ ID No.10, or a DNA molecule that hybridizes with the DNA molecule shown in SEQ ID No.10 under stringent conditions and encodes a protein with the amino acid sequence SEQ ID No.9, or a DNA molecule that shares more than 99%, 95%, 90%, 85%, or 80% homology with the DNA sequence defined by SEQ ID No.10 and encodes a protein with the amino acid sequence SEQ ID No.9.

In the aforementioned methods, the coding gene for Vitreoscilla hemoglobin (VHb) may be a DNA molecule with the nucleotide sequence SEQ ID No.14, or a DNA molecule that hybridizes with the DNA molecule shown in SEQ ID No.14 under stringent conditions and encodes a protein with the amino acid sequence SEQ ID No.13, or a DNA molecule that shares more than 99%, 95%, 90%, 85%, or 80% homology with the DNA sequence defined by SEQ ID No.14 and encodes a protein with the amino acid sequence SEQ ID No.13.

Among the aforementioned coding genes, stringent conditions may be as follows: hybridizing at 50°C in a mixed solution of 7% sodium dodecyl sulfate (SDS), 0.5M NaPO₄, and 1mM EDTA, and rinsing in 50°C, 2×SSC, 0.1% SDS; or hybridizing at 50°C in a mixed solution of 7% SDS, 0.5M NaPO₄, and 1mM EDTA, and rinsing in 50°C, 1×SSC, 0.1% SDS; or hybridizing at 50°C in a mixed solution of 7% SDS, 0.5M NaPO₄, and 1mM EDTA, and rinsing in 50°C, 0.5×SSC, 0.1% SDS; or hybridizing at 50°C in a mixed solution of 7% SDS, 0.5M NaPO₄, and 1mM EDTA, and rinsing in 50°C, 0.1×SSC, 0.1% SDS; or hybridizing at 50°C in a mixed solution of 7% SDS, 0.5M NaPO₄, and 1mM EDTA, and rinsing in 65°C, 0.1×SSC, 0.1% SDS; or hybridizing in a solution of 6×SSC, 0.5% SDS at 65°C, and then washing the membrane once with 2×SSC, 0.1% SDS and once with 1×SSC, 0.1% SDS.

Among the aforementioned coding genes, homology refers to the identity of nucleotide sequences. The identity of nucleotide sequences can be determined using homology search sites on the internet, such as the BLAST webpage on the NCBI homepage. For example, in advanced BLAST 2.1, by using blastp as the program, setting the Expect value to 10, turning off all Filters, using BLOSUM 62 as the Matrix, and setting the Gap existence cost, Per residue gap cost, and Lambda ratio to 11, 1, and 0.85 (default values), respectively, and performing the search, the identity value (%) of a pair of nucleotide sequences can be calculated.

Among the aforementioned coding genes, homology of more than 95% may indicate at least 96%, 97%, or 98% identity. Homology of more than 90% may indicate at least 91%, 92%, 93%, or 94% identity. Homology of more than 85% may indicate at least 86%, 87%, 88%, or 89% identity. Homology of more than 80% may indicate at least 81%, 82%, 83%, or 84% identity.

In the aforementioned methods, the recipient yeast strain may be Pichia pastoris.

In one embodiment of this disclosure, the Pichia pastoris is Pichia pastoris GS115.

This disclosure also claims protection for the engineering strains constructed using the aforementioned methods.

This disclosure also claims protection for the application of the aforementioned engineering strains in preparing feline serum albumin.

This disclosure also claims protection for a method of preparing feline serum albumin.

The method of preparing feline serum albumin claimed by this disclosure may include the following step: fermenting and expressing (culturing) the aforementioned engineering strains to obtain recombinant feline serum albumin from the fermentation broth.

Further, the temperature for fermentation culture is 28-30°C (e.g., 30°C).

Further, the process of fermentation culture includes adding methanol for induced expression. More specifically, methanol is added in batches (e.g., every 12 hours) for induced expression (the methanol content does not exceed 0.5% of the culture medium volume, e.g., 0.5%), and induced expression is carried out for 72-120 hours (e.g., 72 hours).

Further, the process of fermentation culture may also include all or part of the following: aerating and stirring, with the upper and lower limits of the stirring speed controlled at 200 rpm and 1000 rpm, respectively; setting the temperature at 30°C; setting the pH value at 6.0; feeding glycerol to maintain dissolved oxygen at 30%-40%, stopping glycerol feeding when the bacterial cell growth reaches OD600=200, cooling to 28°C, starting methanol feeding, while maintaining dissolved oxygen at 20%-30%, and continuing fermentation for 72 hours.

### BRIEF DESCRIPTION OF THE DRAWINGS

FIG. 1 shows the map of the pPIC9K vector.
FIG. 2 shows the map of the FSA Pichia pastoris expression vector.
FIG. 3 shows the electrophoresis results of shake-flask expression of the strain expressing FSA alone.
FIG. 4 shows the map of the pPICZα vector.
FIG. 5 shows the map of the PDI Pichia pastoris expression vector.
FIG. 6 shows the electrophoresis results of shake-flask expression of the strain co-expressing FSA and PDI.
FIG. 7 shows the map of the pPIC6α vector.
FIG. 8 shows the map of the HAC1 Pichia pastoris expression vector.
FIG. 9 shows the map of the HVb Pichia pastoris expression vector.
FIG. 10 shows the map of the co-expression vector of HAC1 and HVb.
FIG. 11 shows the electrophoresis results of shake-flask expression of the strain co-expressing FSA and co-factors.
FIG. 12 shows the fermentation electrophoresis results of the strain co-expressing VHb, HAC1, rFSA, and PDI.

### DETAILED DESCRIPTION

The following examples are provided to facilitate a better understanding of the present disclosure, but they do not limit the scope of the present disclosure. Unless otherwise specified, the experimental methods in the following examples are conventional methods. Unless otherwise specified, the test materials used in the following examples are purchased from regular biochemical reagent stores. In the quantitative experiments in the following examples, three repeated experiments are set up, and the results are averaged.

### Example 1: High-Efficiency Expression of Recombinant Feline Serum Albumin (rFSA)

### 1. FSA Gene Synthesis and Yeast Expression Vector Construction

The amino acid sequence of feline serum albumin published in the Uniprot database is shown in SEQ ID No.1. The gene sequence of feline serum albumin (SEQ ID No.2) is synthesized according to the optimal codons of Pichia pastoris. The gene synthesis is completed by Suzhou Genewiz Biotechnology Co., Ltd. The following primers are designed to amplify the synthesized gene sequence by PCR using SEQ ID No.2 as the template:
FSA-F: 5'-GAAGAAGGGGTATCTCTCGAGAAAAGAGAAGCTCATCAATCTG-3' (SEQ ID No.3);
FSA-R: 5'-ATAAGAATGCGGCCGCTTAAGCCAAAGCAGCTTGAG-3' (SEQ ID No.4).

The specific conditions are as follows: denaturation at 95°C for 3 minutes; 30 cycles of denaturation at 95°C for 30 seconds, annealing at 52°C for 30 seconds, and extension at 72°C for 1 minute and 30 seconds; followed by extension at 72°C for 5 minutes.

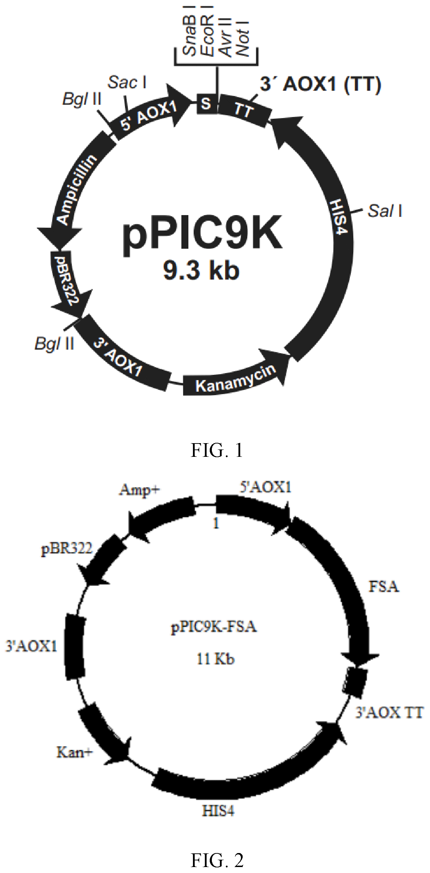

The Pichia pastoris expression vector pPIC9K (FIG. 1) contains the yeast α-factor signal peptide, which can be used for the secretion and expression of the target protein. The vector is double-digested with EcoRI and NotI, and the FSA gene obtained by PCR is used to construct the expression vector pPIC9K-FSA (FIG. 2) using the BM Seamless Cloning Kit (product of Beijing Bomai De Gene Technology Co., Ltd.), and the sequencing is correct.

### 2. Electroporation of the pPIC9K-FSA Expression Vector into Pichia pastoris

In this disclosure, Pichia pastoris GS115 is used as the host strain. The preparation method of the electroporation-competent cells referred to the instruction manual of the pPICZα vector from Invitrogen. The pPIC9K-FSA vector is linearized with SalI, added to 150 µl of GS115 electroporation-competent cells, and electroporated using a Bio-Rad MicroPulser electroporator (2 mm cuvette, 2000 V, 5 ms). The transformed products are spread on MD solid medium (formula: 1.34% amino acid-free yeast nitrogen base, 4×10⁻⁵% biotin, 2% glucose, 2% agar powder; each % represents g/100 ml) and cultured at 30°C.

### 3. Obtainment of rFSA-Expressing Strains

The recombinant strains grown on the MD plates are transferred to YPD solid medium (formula: 2% peptone, 1% yeast extract, 2% glucose, 2% agar powder; each % represents g/100 ml) containing 2 mg/ml geneticin (G418) and cultured at 30°C for 48 hours for antibiotic enrichment screening. The strains grown after the first antibiotic enrichment are transferred to YPD solid medium containing 4 mg/ml geneticin (G418) and cultured at 30°C for 48 hours for a second antibiotic enrichment, thereby obtaining the rFSA-expressing strain GS115-FSA with multiple copies integrated.

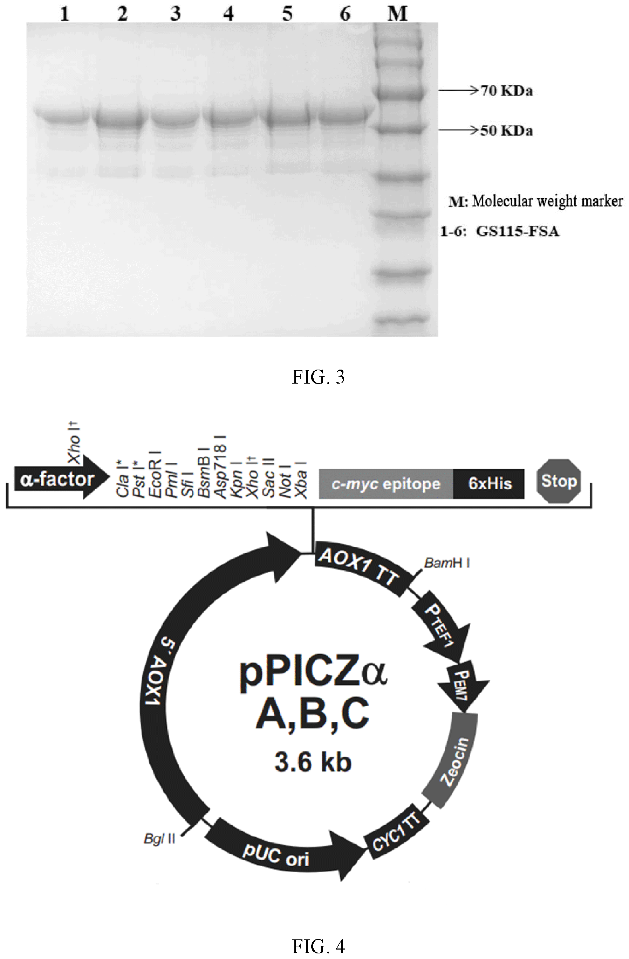

A single colony of GS115-FSA is selected and inoculated into 10 ml of YPD liquid medium (formula: 2% peptone, 1% yeast extract, 2% glucose; each % represents g/100 ml) and cultured at 30°C for 24 hours. The cells are collected by centrifugation and transferred to 20 ml of YP liquid medium (formula: 2% peptone, 1% yeast extract, 0.5% methanol; peptone and yeast extract are mass-volume ratios (i.e., % represents g/100 ml), and methanol is a volume percentage) for induction and expression for 72 hours, with 0.5% (volume percentage) methanol added every 12 hours. After induction, the culture supernatant is taken for SDS-PAGE electrophoresis detection. The results are shown in FIG. 3, and a high-expressing FSA strain is obtained.

### 4. PDI Gene Synthesis and Yeast Expression Vector Construction

The amino acid sequence of Pichia pastoris protein disulfide isomerase (PDI) published in the Uniprot database is shown in SEQ ID No.5. The PDI gene sequence (SEQ ID No.6) is synthesized according to the optimal codons of Pichia pastoris. The gene synthesis is completed by Suzhou Genewiz Biotechnology Co., Ltd. The following primers are designed to amplify the synthesized gene sequence by PCR using SEQ ID No.6 as the template:
PDI-F: 5'-GAAGAAGGGGTATCTCTCGAGAAAAGATCTGACCAAGAAGCTAT-3' (SEQ ID No.7);
PDI-R: 5'-GTTCTAGAAAGCTGGCGGCCGCTTACAATTCGTCGTGAGC-3' (SEQ ID No.8).

The specific conditions are as follows: denaturation at 95°C for 3 minutes; 30 cycles of denaturation at 95°C for 30 seconds, annealing at 52°C for 30 seconds, and extension at 72°C for 1 minute; followed by extension at 72°C for 5 minutes.

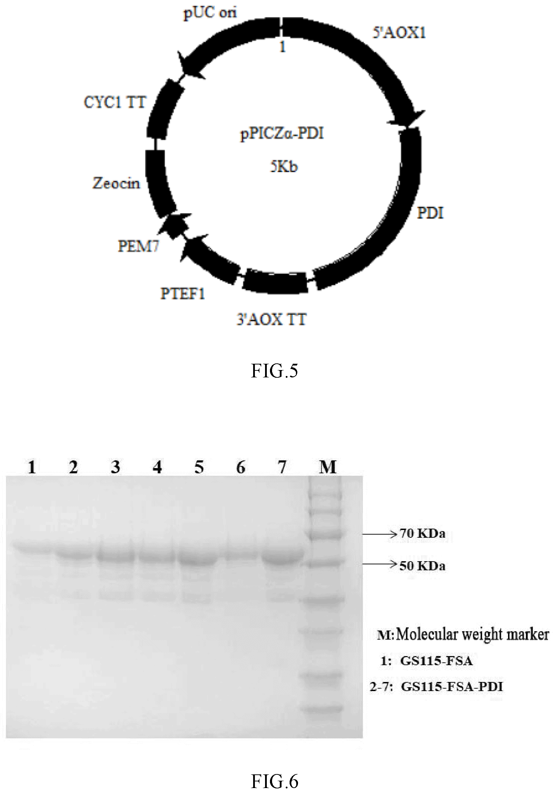

The Pichia pastoris expression vector pPICZα (FIG. 4) contains the bleomycin (Zeocin) resistance gene, which can provide an additional screening marker. The vector is double-digested with XhoI and NotI, and the PDI gene obtained by PCR is used to construct the expression vector pPICZα-PDI (FIG. 5) using the BM Seamless Cloning Kit (product of Beijing Bomai De Gene Technology Co., Ltd.), and the sequencing is correct.

### 5. Electroporation of the pPICZα-PDI Expression Vector into Pichia pastoris

The GS115-FSA strain obtained in step 3 is used as the host strain to prepare the electroporation-competent cells. The pPICZα-PDI vector is linearized with SacI, added to 150 µl of GS115-FSA electroporation-competent cells, and electroporated using a Bio-Rad MicroPulser electroporator (2 mm cuvette, 2000 V, 5 ms). The transformed products are spread on YPD solid medium (formula: 2% peptone, 1% yeast extract, 2% glucose, 100 µg/ml Zeocin, 2% agar powder; each % represents g/100 ml) and cultured at 30°C.

### 6. Screening of rFSA and PDI Co-Expressing Strains

The recombinant strains grown on the transformation plates are transferred to YPD solid medium (formula: 2% peptone, 1% yeast extract, 2% glucose, 2% agar powder; each % represents g/100 ml) containing 2 mg/ml bleomycin (Zeocin) and cultured at 30°C for 48 hours for antibiotic enrichment screening. The strains grown after the first antibiotic enrichment are transferred to YPD solid medium containing 4 mg/ml bleomycin (Zeocin) and cultured at 30°C for 48 hours for a second antibiotic enrichment, thereby obtaining the rFSA-expressing strain GS115-FSA-PDI with multiple copies of the PDI gene integrated.

A single colony of GS115-FSA-PDI is selected and inoculated into 10 ml of YPD liquid medium (formula: 2% peptone, 1% yeast extract, 2% glucose; each % represents g/100 ml) and cultured at 30°C for 24 hours. The cells are collected by centrifugation and transferred to 20 ml of YP liquid medium (formula: 2% peptone, 1% yeast extract, 0.5% methanol; peptone and yeast extract are mass-volume ratios (i.e., % represents g/100 ml), and methanol is a volume percentage) for induction and expression for 72 hours, with 0.5% (volume percentage) methanol added every 12 hours. After induction, the culture supernatant is taken for SDS-PAGE electrophoresis detection shown in FIG. 6. The FSA expression level of the rFSA and PDI co-expressing strain is significantly higher than that of the original strain.

### 7. HAC1 Gene Synthesis and Yeast Expression Vector Construction

The amino acid sequence of Pichia pastoris HAC1 published in the Uniprot database is shown in SEQ ID No.9. The HAC1 gene sequence (SEQ ID No.10) is synthesized according to the optimal codons of Pichia pastoris. The gene synthesis is completed by Suzhou Genewiz Biotechnology Co., Ltd. The following primers are designed to amplify the synthesized gene sequence by PCR using SEQ ID No.10 as the template:
HAC1-F: 5'-CAACTAATTATTCGAAACGATGCCAGTTGACTCTTC-3' (SEQ ID No.11);
HAC1-R: 5'-GTTCTAGAAAGCTGGCGGCCGCTTATCTGATAGCGATACAAG-3' (SEQ ID No.12).

The specific conditions are as follows: denaturation at 95°C for 3 minutes; 30 cycles of denaturation at 95°C for 30 seconds, annealing at 52°C for 30 seconds, and extension at 72°C for 1 minute; followed by extension at 72°C for 5 minutes.

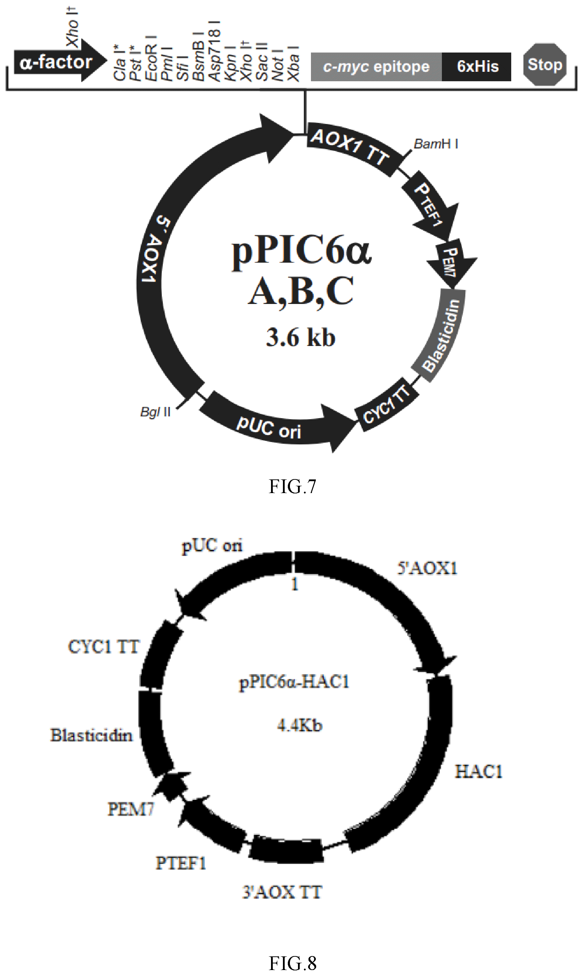

The Pichia pastoris expression vector pPIC6α shown in FIG. 7 contains the blasticidin resistance gene, which can provide an additional screening marker. The vector is double-digested with AusII and NotI, and the HAC1 gene obtained by PCR is used to construct the expression vector pPIC6α-HAC1 (FIG. 8) using the BM Seamless Cloning Kit (product of Beijing Bomai De Gene Technology Co., Ltd.), and the sequencing is correct.

### 8. VHb Gene Synthesis and Yeast Expression Vector Construction

The amino acid sequence of Vitreoscilla hemoglobin (VHb) published in the Uniprot database is shown in SEQ ID No.13. The VHb gene sequence (SEQ ID No.14) is synthesized according to the optimal codons of Pichia pastoris. The gene synthesis is completed by Suzhou Genewiz Biotechnology Co., Ltd. The following primers are designed to amplify the synthesized gene sequence by PCR using SEQ ID No.14 as the template:
VHb-F1: 5'-CAACTAATTATTCGAAACGATGTTGGACCAACAAACT-3' (SEQ ID No.15);
VHb-R1: 5'-GTTCTAGAAAGCTGGCGGCCGCTTATTCAACAGCTTGAGCG-3' (SEQ ID No.16).

The specific conditions are as follows: denaturation at 95°C for 3 minutes; 30 cycles of denaturation at 95°C for 30 seconds, annealing at 52°C for 30 seconds, and extension at 72°C for 30 seconds; followed by extension at 72°C for 5 minutes.

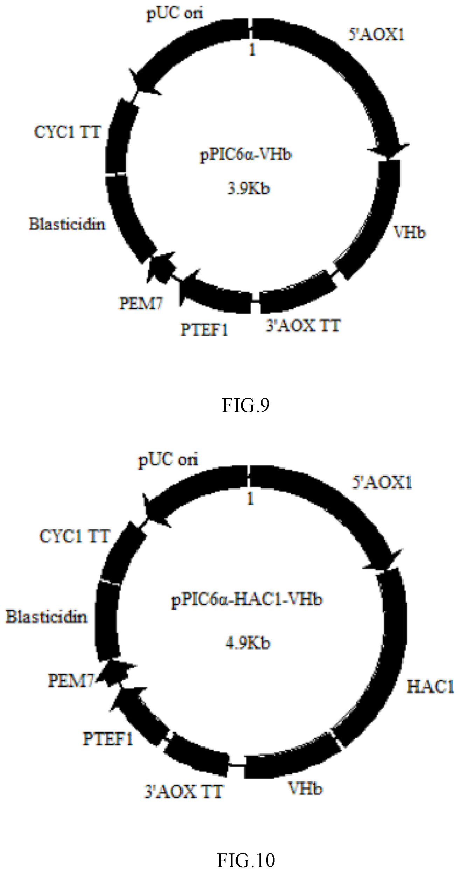

The pPIC6α vector is double-digested with AusII and NotI, and the VHb gene obtained by PCR is used to construct the expression vector pPIC6α-VHb (FIG. 9) using the BM Seamless Cloning Kit (product of Beijing Bomai De Gene Technology Co., Ltd.), and the sequencing is correct.

### 9. Construction of the HAC1 and VHb Dual Expression Vector

The following primers are designed to amplify the VHb gene sequence by PCR using SEQ ID No.14 as the template:
VHb-F2: 
5’-CTTGTATCGCTATCAGATAAGCGGCCGCCAACTAATTATTCGAAACGATGTTGGACC
AACAAACT-3’ (SEQ ID No.17);
VHb-R2: 5'-CGCTCAAGCTGTTGAATAATCTAGAACAAAAACTCATC-3' (SEQ ID No.18).

The specific conditions are as follows: denaturation at 95°C for 3 minutes; 30 cycles of denaturation at 95°C for 30 seconds, annealing at 52°C for 30 seconds, and extension at 72°C for 30 seconds; followed by extension at 72°C for 5 minutes.

The previously constructed pPIC6α-HAC1 vector is double-digested with NotI and XbaI, and the VHb gene obtained by PCR is used to construct the expression vector pPIC6α-HAC1-VHb (FIG. 10) using the BM Seamless Cloning Kit (product of Beijing Bomai De Gene Technology Co., Ltd.), and the sequencing is correct.

### 10. Construction and Screening of HAC1, rFSA, and PDI Co-Expressing Strains

The GS115-FSA-PDI strain obtained in step 6 is used as the host strain to prepare the electroporation-competent cells. The pPIC6α-HAC1 vector is linearized with SacI, added to 150 µl of GS115-FSA-PDI electroporation-competent cells, and electroporated using a Bio-Rad MicroPulser electroporator (2 mm cuvette, 2000 V, 5 ms). The transformed products are spread on YPD solid medium (formula: 2% peptone, 1% yeast extract, 2% glucose, 100 µg/ml blasticidin, 2% agar powder; each % represents g/100 ml) and cultured at 30°C.

The recombinant strains grown on the transformation plates are transferred to YPD solid medium (formula: 2% peptone, 1% yeast extract, 2% glucose, 2% agar powder; each % represents g/100 ml) containing 2 mg/ml blasticidin and cultured at 30°C for 48 hours for antibiotic enrichment screening. The strains grown after the first antibiotic enrichment are transferred to YPD solid medium containing 4 mg/ml blasticidin and cultured at 30°C for 48 hours for a second antibiotic enrichment, thereby obtaining the rFSA-expressing strain GS115-FSA-PDI-HAC1 with multiple copies of the HAC1 gene integrated.

### 11. Construction and Screening of VHb, rFSA, and PDI Co-Expressing Strains

The GS115-FSA-PDI strain obtained in step 6 is used as the host strain to prepare the electroporation-competent cells. The pPIC6α-VHb vector is linearized with SacI, added to 150 µl of GS115-FSA-PDI electroporation-competent cells, and electroporated using a Bio-Rad MicroPulser electroporator (2 mm cuvette, 2000 V, 5 ms). The transformed products are spread on YPD solid medium (formula: 2% peptone, 1% yeast extract, 2% glucose, 100 µg/ml blasticidin, 2% agar powder; each % represents g/100 ml) and cultured at 30°C.

The recombinant strains grown on the transformation plates are transferred to YPD solid medium (formula: 2% peptone, 1% yeast extract, 2% glucose, 2% agar powder; each % represents g/100 ml) containing 2 mg/ml blasticidin and cultured at 30°C for 48 hours for antibiotic enrichment screening. The strains grown after the first antibiotic enrichment are transferred to YPD solid medium containing 4 mg/ml blasticidin and cultured at 30°C for 48 hours for a second antibiotic enrichment, thereby obtaining the rFSA-expressing strain GS115-FSA-PDI-VHb with multiple copies of the VHb gene integrated.

### 12. Construction and Screening of VHb, HAC1, rFSA, and PDI Co-Expressing Strains

The GS115-FSA-PDI strain obtained in step 6 is used as the host strain to prepare the electroporation-competent cells. The pPIC6α-HAC1-VHb vector is linearized with SacI, added to 150 µl of GS115-FSA-PDI electroporation-competent cells, and electroporated using a Bio-Rad MicroPulser electroporator (2 mm cuvette, 2000 V, 5 ms). The transformed products are spread on YPD solid medium (formula: 2% peptone, 1% yeast extract, 2% glucose, 100 µg/ml blasticidin, 2% agar powder; each % represents g/100 ml) and cultured at 30°C.

The recombinant strains grown on the transformation plates are transferred to YPD solid medium (formula: 2% peptone, 1% yeast extract, 2% glucose, 2% agar powder; each % represents g/100 ml) containing 2 mg/ml blasticidin and cultured at 30°C for 48 hours for antibiotic enrichment screening. The strains grown after the first antibiotic enrichment are transferred to YPD solid medium containing 4 mg/ml blasticidin and cultured at 30°C for 48 hours for a second antibiotic enrichment, thereby obtaining the rFSA-expressing strain GS115-FSA-PDI-HAC1-VHb with multiple copies of the HAC1 and VHb genes integrated.

### 13. Shake-Flask Expression of rFSA and Cofactor Co-Expressing Strains

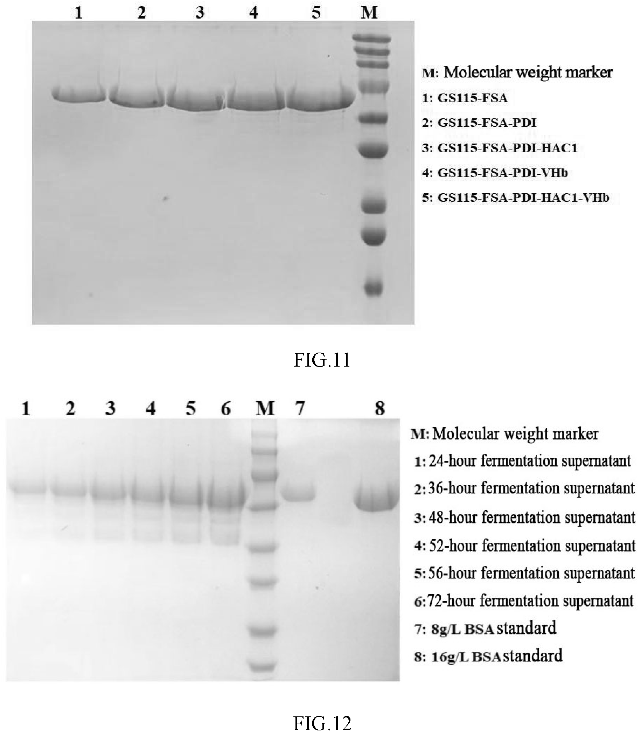

Single colonies of the GS115-FSA-PDI-HAC1, GS115-FSA-PDI-VHb, and GS115-FSA-PDI-HAC1-VHb strains obtained in the above steps are selected and inoculated into 10 ml of YPD liquid medium (formula: 2% peptone, 1% yeast extract, 2% glucose; each % represents g/100 ml) and cultured at 30°C for 24 hours. The cells are collected by centrifugation and transferred to 20 ml of YP liquid medium (formula: 2% peptone, 1% yeast extract, 0.5% methanol; peptone and yeast extract are mass-volume ratios (i.e., % represents g/100 ml), and methanol is a volume percentage) for induction and expression for 72 hours, with 0.5% methanol (volume percentage) added every 12 hours. After induction, the culture supernatant is taken for SDS-PAGE electrophoresis detection. The results are shown in FIG. 11. According to the calculation using the Quantity One protein imaging software, the GS115-FSA-PDI-HAC1-VHb strain had the highest FSA expression level, and the FSA expression levels of all the cofactor co-expressing strains are significantly higher than that of the original strain.

### 14. Fermentation of VHb, HAC1, rFSA, and PDI Co-Expressing Strains

The high FSA-expressing strain GS115-FSA-PDI-HAC1-VHb obtained in step 13 is used as the starting strain for fermentation in a 5 L fermenter.

Seed medium: 2% peptone (mass-volume ratio, i.e., % represents g/100 ml), 1% yeast extract (mass-volume ratio, i.e., % represents g/100 ml), 1% glycerol (volume percentage).

Fermentation medium: 1 L contains 40 g of glycerol, 26.7 mL of H₃PO₄ (85% concentration), 0.93 g of CaSO₄, 18.2 g of K₂SO₄, 14.9 g of MgSO₄·7H₂O, 4.13 g of KOH, and 1.6 mL of biotin solution (0.2 g/L).

Feed supplements: ammonia water (automatically added according to the set pH value), glycerol, and methanol (automatically added according to a dissolved oxygen value of 20-30%).

### (1) Seed Culture

A single colony of the GS115-FSA-PDI-HAC1-VHb strain screened in step 13 is inoculated into 500 mL of seed medium and cultured in a shaker at 30°C for 24 hours. After culture, all the bacterial culture is inoculated into 4.5 L of fermentation medium.

### (2) Fermentation Culture

Fermentation is carried out in a 5 L fermenter with aeration and stirring. The upper and lower limits of the stirring speed are controlled at 200 rpm and 1000 rpm, respectively. The temperature is set at 30°C, the pH value is set at 6.0, glycerol is added to maintain the dissolved oxygen at 30%-40%. When the cell growth reached an OD₆₀₀ of 200, glycerol addition is stopped, the temperature is lowered to 28°C, and methanol addition is started to maintain the dissolved oxygen at 20%-30%. Fermentation is continued for 72 hours.

The FSA expression level in the culture supernatant is analyzed by electrophoresis at different fermentation time points. BSA standards (set at two concentration gradients of 8 g/L and 16 g/L) are used as controls. The electrophoresis results are shown in FIG. 12. According to the calculation using the Quantity One protein imaging software, the FSA expression level in the 72-hour fermentation supernatant is consistent with the electrophoresis result of the 16 g/L BSA standard (i.e., the yield of this disclosure could reach approximately 16 g/L). This disclosure lays the foundation for the large-scale industrial production of FSA.

### Industrial Application

The engineered strain of this disclosure can efficiently express rFSA. Compared with the direct expression of rFSA alone, the expression level of rFSA in the co-expressing strains is significantly increased, up to 16 g/L. This disclosure lays the foundation for the large-scale industrial production of rFSA.

## Claims

1. A method for constructing an engineered strain for recombinant expression of feline serum albumin, comprising the following step (A):
(A) co-expressing feline serum albumin, protein disulfide isomerase, and regulatory factor HAC1 in a recipient yeast strain, and the resulting strain is named engineered strain 1; the engineered strain 1 is an engineered strain for recombinant expression of feline serum albumin.

2. A method for constructing an engineered strain for expressing recombinant feline serum albumin, comprising the following step (B):
(B) co-expressing feline serum albumin, protein disulfide isomerase, and vitreoscilla hemoglobin in a recipient yeast strain, and the resulting strain is named engineered strain 2; the engineered strain 2 is an engineered strain for recombinant expression of feline serum albumin.

3. A method for constructing an engineered strain for recombinant expression of feline serum albumin, comprising the following step (C):
(C) co-expressing feline serum albumin, protein disulfide isomerase, regulatory factor HAC1, and vitreoscilla hemoglobin in a recipient yeast strain, and the resulting strain is named engineered strain 3; the engineered strain 3 is an engineered strain for recombinant expression of feline serum albumin.

4. The method according to claim 1, wherein, the step (A) is to introduce the coding gene of the serum albumin, the coding gene of the protein disulfide isomerase, and the coding gene of the regulatory factor HAC1 into the recipient yeast strain, and the resulting strain is the engineered strain 1.

5. The method according to claim 2, wherein, the step (B) is to introduce the coding gene of the serum albumin, the coding gene of the protein disulfide isomerase, and the coding gene of the vitreoscilla hemoglobin into the recipient yeast strain, and the resulting strain is the engineered strain 2.

6. The method according to claim 3, wherein, the step (C) is to introduce the coding gene of the serum albumin, the coding gene of the protein disulfide isomerase, the coding gene of the regulatory factor HAC1, and the coding gene of the vitreoscilla hemoglobin into the recipient yeast strain, and the resulting strain is the engineered strain 3.

7. The method according to any one of claims 1-6, wherein, the feline serum albumin is a protein with an amino acid sequence of SEQ ID No.1, or a protein with one or several amino acid residue substitutions and/or deletions and/or additions compared to SEQ ID No.1 and having the same function, or a protein with more than 99%, more than 95%, more than 90%, more than 85%, or more than 80% homology to SEQ ID No.1 and having the same function, or a fusion protein obtained by attaching a tag to the N-terminus and/or C-terminus of the protein with the amino acid sequence of SEQ ID No.1.

8. The method according to any one of claims 1-7, wherein, the protein disulfide isomerase is a protein with the amino acid sequence of SEQ ID No.5, or a protein with one or several amino acid residue substitutions and/or deletions and/or additions compared to SEQ ID No.5 and having the same function, or a protein with more than 99%, more than 95%, more than 90%, more than 85%, or more than 80% homology to SEQ ID No.5 and having the same function, or a fusion protein obtained by attaching a tag to the N-terminus and/or C-terminus of the protein with the amino acid sequence of SEQ ID No.5.

9. The method according to any one of claims 1-8, wherein, the regulatory factor HAC1 is a protein with the amino acid sequence of SEQ ID No.9, or a protein with one or several amino acid residue substitutions and/or deletions and/or additions compared to SEQ ID No.9 and having the same function, or a protein with more than 99%, more than 95%, more than 90%, more than 85%, or more than 80% homology to SEQ ID No.9 and having the same function, or a fusion protein obtained by attaching a tag to the N-terminus and/or C-terminus of the protein with the amino acid sequence of SEQ ID No.9.

10. The method according to any one of claims 1-9, wherein, the Vitreoscilla hemoglobin is a protein with the amino acid sequence of SEQ ID No.13, or a protein with one or several amino acid residue substitutions and/or deletions and/or additions compared to SEQ ID No.13 and having the same function, or a protein with more than 99%, more than 95%, more than 90%, more than 85%, or more than 80% homology to SEQ ID No.13 and having the same function, or a fusion protein obtained by attaching a tag to the N-terminus and/or C-terminus of the protein with the amino acid sequence of SEQ ID No.13.

11. The method according to any one of claims 1-10, wherein, the coding gene of serum albumin is a DNA molecule with the nucleotide sequence of SEQ ID No.2, or a DNA molecule that hybridizes to the DNA molecule shown in SEQ ID No.2 under stringent conditions and encodes the protein with the amino acid sequence of SEQ ID No.1, or a DNA molecule with more than 99%, more than 95%, more than 90%, more than 85%, or more than 80% homology to the DNA sequence defined by SEQ ID No.2 and encodes the protein with the amino acid sequence of SEQ ID No.1.

12. The method according to any one of claims 1-11, wherein, the coding gene of protein disulfide isomerase is a DNA molecule with the nucleotide sequence of SEQ ID No.6, or a DNA molecule that hybridizes to the DNA molecule shown in SEQ ID No.6 under stringent conditions and encodes the protein with the amino acid sequence of SEQ ID No.5, or a DNA molecule with more than 99%, more than 95%, more than 90%, more than 85%, or more than 80% homology to the DNA sequence defined by SEQ ID No.6 and encodes the protein with the amino acid sequence of SEQ ID No.5.

13. The method according to any one of claims 1-12, wherein, the coding gene of regulatory factor HAC1 is a DNA molecule with the nucleotide sequence of SEQ ID No.10, or a DNA molecule that hybridizes to the DNA molecule shown in SEQ ID No.10 under stringent conditions and encodes the protein with the amino acid sequence of SEQ ID No.9, or a DNA molecule with more than 99%, more than 95%, more than 90%, more than 85%, or more than 80% homology to the DNA sequence defined by SEQ ID No.10 and encodes the protein with the amino acid sequence of SEQ ID No.9.

14. The method according to any one of claims 1-13, wherein, the coding gene of vitreoscilla hemoglobin is a DNA molecule with the nucleotide sequence of SEQ ID No.14, or a DNA molecule that hybridizes to the DNA molecule shown in SEQ ID No.14 under stringent conditions and encodes the protein with the amino acid sequence of SEQ ID No.13, or a DNA molecule with more than 99%, more than 95%, more than 90%, more than 85%, or more than 80% homology to the DNA sequence defined by SEQ ID No.14 and encodes the protein with the amino acid sequence of SEQ ID No.13.

15. The method according to any one of claims 1-14, wherein, the recipient yeast strain is pichia pastoris.

16. The method according to claim 15, wherein, the pichia pastoris is pichia pastoris GS115.

17. An engineered strain constructed by the method according to any one of claims 1-16.

18. The use of the engineered strain according to claim 17 in the preparation of feline serum albumin.

19. A method for preparing feline serum albumin, comprising the following steps: fermenting and expressing the engineered strain according to claim 17, and obtaining feline serum albumin from the fermentation broth.
